# EUROPEAN PATENT APPLICATION

(11) **EP 4 531 052 A1**
(43) Date of publication of application: **02.04.2025**
(21) Application number: 23200442.4
(22) Date of filing: 28.09.2023
(51) Int. Cl.: G16H 20/40, G16H 50/20, G16H 50/30, G16H 50/50, G16H 50/70, G16H 70/20

(54) **EPREDICTING MEDICAL PROCEDURE OUTCOMES**

(71) Applicant: Koninklijke Philips N.V., 5656 AG Eindhoven (NL)
(72) Inventor: GAYET, Maudy, Eindhoven (NL); VYDHYAR VEETTIL, Ayshwariya, Eindhoven (NL); SCHUURKAMP, Gertjan Laurens, 5656AG Eindhoven (NL); VOS, Pieter C., Eindhoven (NL); DE BECKER, Jan, Eindhoven (NL); HOFFMANN, Ralf Dieter, Eindhoven (NL); HEIJMAN, Edwin, Eindhoven (NL)
(74) Representative: Philips Intellectual Property & Standards

(57) **Abstract**

A mechanism for predicting an outcome of user-defined medical procedure to be performed on a medical subject. Subject information and user-defined procedural information is processed to predict one or more outcomes of the user-defined medical procedure, which is identified in the user-defined procedural information.

## Description

### FIELD OF THE INVENTION

The present invention relates to the field of predicting the outcome of performing a medical procedure on a medical subject.

### BACKGROUND OF THE INVENTION

A significant problem facing clinical workers, such as physicians, is to make treatment decisions for a medical subject. Presently, it is common for an interdisciplinary group of clinicians to assemble and discuss potential treatment options for a medical subject, selecting the perceived best option based a review of available subject information and using their experience to make the clinical decision as to how to treat the medical subject.

A complicating factor for making a clinical decision is inconsistency in accessing data sources for the subject information. In particular, there is a wide diversity of data sources, which hinders a direct analysis of patient information by a clinician.

There is an ongoing desire to improve the level of support provided to a clinician in making a clinical decision.

### SUMMARY OF THE INVENTION

According to examples in accordance with an aspect of the invention, there is provided a computer-implemented method of generating at least one predicted outcome of a medical procedure on a medical subject.

The computer-implemented method comprises: obtaining subject information that identifies one or more characteristics of the medical subject; obtaining user-defined procedural information identifying a potential medical procedure to be performed on the medical subject, wherein the user-defined procedural information is defined by a user interacting with a user interface; and processing the subject information and the user-defined procedural information using one or more risk models to generate at least one predicted outcome of performing the identified potential medical procedure on the medical subject.

The present disclosure proposes to generate one or more predicted outcomes for the performance of one or more user-specified medical procedures performed on a subject defined by their subject information. This approach can advantageously aid a clinician into understanding the likely prognosis for particular treatment decisions, allowing improved understanding of the medical subject's condition and/or risk. This aids in making a clinical decision for how best to treat the medical subject.

It is noted that a predicted outcome of performing a medical procedure is considered to represent an inherent property of the clinical subject, as it changes responsive to an internal state of the medical subject. The predicted outcome(s) would actively assist a clinician to make a clinical decision as to how to treat the medical subject.

The subject information and the user-defined procedural information is information obtained and/or defined before or without the medical procedure actually taking place. Thus, the generation of the at least one predicted outcome can be performed in advance of any medical procedure, to aid in the selection or determination of the medical procedure to perform.

In this way, only pre-operative or pre-procedural information is processed in order to predict an outcome of actually performing the medical procedure.

Thus, the present disclosure provides a method for predicting an outcome of a medical procedure that has not yet been performed, and indeed, may not be performed. Thus, the medical procedure is only a potential medical procedure to be performed, rather than an actually performed medical procedure.

The user-defined procedural information may identify at least one potential surgical procedure to be performed on the medical subject. Surgical procedures carry a high level of inherent risk, such that it would be preferable to understand the probable outcomes of a such surgical procedures before committing to a surgical treatment plan. In particular, a wide variety of potential surgical procedures exist, with the outcomes of different surgical procedures varying significantly depending upon medical subject information. The proposed approach reduces or avoids a likelihood of a clinician overlooking or misunderstanding the outcome of a treatment option for a particular medical subject.

The user-defined procedural information may identify at least one potential prostatectomy surgical procedure to be performed on the medical subject.

In some examples, the user-defined procedural information identifies at least one potential nerve-sparing technique to be performed on the medical subject.

In some examples, the at least one predicted outcome comprises one or more risk measures, each risk measure being a measure of predicted risk to the medical subject. Risk measures are an important consideration in making a clinical decision for a medical subject, such that generation of such risk measures is particularly advantageous.

The one or more risk measures may comprise a measure of patient-specific risk; oncological risk; and/or risk of procedural complications.

In some examples, the at least one predicted outcome comprises one or more predicted functional outcomes for the medical subject.

The at least one predicted outcome comprises one or more recovery probabilities, each recovery probability identifying a probability of recovery of a biological function of the medical subject.

The precise biological function(s) may depend upon the target oncology and/or potential procedure(s) to be performed on the medical subject. For instance, if the potential procedure(s) are designed for prostatectomy, then suitable biological functions include: continence function; rectal function; and/or potency function.

The least one predicted outcome may comprise one or more survival probabilities, each survival probability identifying a probability of survival of the medical subject, if the at least one potential procedure is performed on the medical subject, for a predetermined period of time after the at least one potential procedure is performed on the medical subject.

The at least one predicted outcome comprises one or more reoccurrence probabilities, each reoccurrence probability identifying a probability of a medical condition or pathology reoccurring, if the at least one potential procedure is performed on the medical subject, within a predefined period of time after the at least one potential procedure is performed on the medical subject.

In some examples, the at one risk models uses one or more clinical guidelines to generate the at least one predicted outcome. This approach increases the accuracy of the predicted outcomes, as the clinical guidelines are defined by expert knowledge and/or research. This approach facilitates the automated integration of clinical guidelines into the treatment decision procedure, significantly reducing a likelihood of an inappropriate (e.g., clinically adverse) clinical decision being made.

The at one risk model may use one or more machine-learning methods to generate the at least one predicted outcome. The use of a machine-learning method allows for hidden or unknown relationships between medical subject information, medical procedures and clinical outcomes to be automatically included in the prediction of clinical outcomes. This can significantly increase the accuracy of the predicted outcome(s), providing more accurate information on the condition of the subject.

The method may further comprise controlling a user interface to provide a visual representation of the at least one predicted outcome.

There is also proposed a computer program product comprising computer program code means which, when executed on a computing device having a processing system, cause the processing system to perform all of the steps of any herein disclosed method.

There is also proposed a processing system configured to generate at least one predicted outcome of a medical procedure on a medical subject. The processing system is configured to: obtain subject information that identifies one or more characteristics of the medical subject; obtain user-defined procedural information identifying a potential medical procedure to be performed on the medical subject, wherein the user-defined procedural information is defined by a user interacting with a user interface; and process the subject information and the user-defined procedural information using one or more risk models to generate at least one predicted outcome of performing the identified potential medical procedure on the medical subject.

The skilled person would be readily capable of adapting any herein proposed processing system to perform the functions of any herein disclosed method, and vice versa.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flowchart illustrating a method according to a proposed approach;
Fig. 2 illustrates an approach for obtaining user-defined procedural information;
Fig. 3 illustrates example predicted outcomes;
Fig. 4 illustrates a processing system; and
Fig. 5 illustrates a user interface system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for predicting an outcome of user-defined medical procedure to be performed on a medical subject. Subject information and user-defined procedural information is processed to predict one or more outcomes of the user-defined medical procedure, which is identified in the user-defined procedural information.

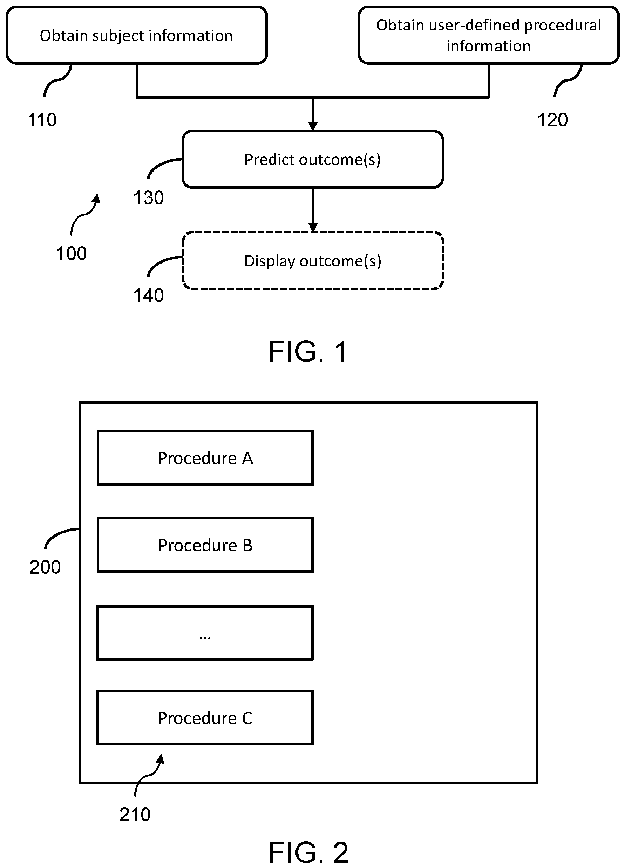

Fig. 1 is a flowchart illustrating a proposed computer-implemented method 100. The computer-implemented method is configured to generate or predict at least one predicted outcome of a medical procedure on a medical subject.

The computer-implemented method 100 comprises a step 110 of obtaining subject information that identifies one or more characteristics of the medical subject. The subject information may, for instance, be obtained from a database or storage memory - e.g., storing an electronic medical record (EMR) of the medical subject.

A wide variety of examples for the one or more characteristics of the medical subject are considered. Example characteristics of the medical subject include: (clinically-relevant) demographic characteristics (e.g., age, gender, weight, fitness level and so on); physiological or biomedical characteristics (e.g., PSA blood markers, average heart rate, average respiratory rate and so on); diagnostic characteristics (e.g., identifying any conditions, diseases and/or pathologies of which the medical subject has been diagnosed and/or information about such conditions, diseases and/or pathologies (e.g., tumor size)); and/or genetic characteristics (e.g., family history information and/or genetic markers).

The computer-implemented method 100 also comprise a step 120 of obtaining user-defined procedural information identifying a potential medical procedure to be performed on the medical subject. The user-defined procedural information is defined by a user interacting with a user interface. The user-defined procedural information may be obtained directly from the user interface and/or from a memory or storage unit configured to store information provided by a user at the user interface.

The user-defined procedural information may thereby indicate or identify information about a user-defined (i.e., user-desired) potential medical procedure to be performed on the subject. This may be indicated by a user (e.g., a clinician or the like) interacting with the user interface to identify a potential medical procedure that could be performed on the subject. The potential medical procedure will typically be a medical procedure designed to treat or otherwise address a clinical pathology of the medical subject.

The method 100 also comprises a step 130 of processing the subject information and the user-defined procedural information using one or more risk models to generate at least one predicted outcome of performing the identified potential medical procedure on the medical subject.

Each risk model aims to map the subject information and user-defined procedural information to a predicted outcome. In particular, a different risk model may produce a different predicted outcome (e.g., a value or values for a different type of predicted outcome).

At least one risk model may, for instance, be formed of a decision tree that uses the subject information and user-defined procedural information to determine a predicted outcome. As another example, at least one risk model may be formed as a machine-learning method configured to process the subject information and user-defined procedural information to generate a predicted outcome.

In some examples, at least one risk model uses clinical guidelines (e.g., stored in a clinical guideline database) to predict outcomes. The clinical guidelines may, for instance, map or associate particular combinations of medical procedure(s) and subject information to predicted outcomes. By way of example, a clinical guideline may indicate that a risk of complications is high for an individual over a certain age undergoing a complex surgical procedure. Other examples will be readily apparent to the skilled person.

The method 100 may further comprise a step 140 of controlling a user interface to provide a visual representation of the at least one predicted outcome. This advantageously provides information to a user or clinician about a prognosis for a particular indicated set of potential medical procedures. This provides the clinician with information that aids them in making a clinical decision on how (best) to treat the medical subject. Thus, the displayed information contributes to a clinical decision support system (CDSS).

Fig. 2 illustrates one approach for obtaining user-defined procedural information.

A visual representation 210 of a set of possible medical procedures may be provided at the user interface 200. The set of possible medical procedures options may, for instance, represent treatment options for a particular pathology and/or condition, e.g., for the treatment of prostate cancer or the like. The illustrated example identifies a set of possible medical procedures for the treatment of prostate cancer, e.g., including at least one possible prostatectomy surgical procedure and/or post-surgery treatments.

In some examples, the set of possible medical procedures is predefined. Thus, the visual representation may be designed for a particular pathology and/or condition. As an example, the visual representation may form part of a dashboard or program designed for treating a particular pathology and/or condition.

In some examples, a set of possible medical procedures may be selected from a larger pool of possible medical procedures based on diagnostic information of the medical subject. Thus, diagnostic information - identifying one or more pathologies, conditions and/or diseases of the medical subject - may be processed to identify the set of possible medical procedures for treating the identified one or more pathologies, conditions and/or diseases. This provides a user interface that updates for the particular pathology of the medical subject.

A user of the user interface, such as a clinician, may interact with the visual representation 210 to identify or indicate the potential medical procedure(s) to be performed on the medical subject. In particular, the user may identify a sub-set (e.g., some or all) of the set of possible medical procedures as the potential medical procedures to be performed. The identified potential medical procedure(s) form the user-defined procedural information. Put another way, the possible medical procedure(s) selected by the user define the potential medical procedure(s) to be performed on the subject.

Approaches for interacting with a display of information to select a subset of the information are widely known in the art, and may be employed in embodiments. This may include, for instance, selecting (e.g., clicking or tapping) on the relevant possible medical procedure(s).

Embodiments are particularly advantageous when the user-defined procedural information identifies at least one potential prostatectomy surgical procedure to be performed on the medical subject. This is because the outcome of performing such surgical procedures on a medical subject can vary significantly.

In preferred examples, the user-defined procedural information identifies at least one potential nerve-sparing technique to be performed on the medical subject. A nerve-sparing technique, also known as a nerve-sparing prostatectomy, is a minimally invasive technique for treating prostate cancer. It has been recognized that the outcomes for different types of nerve-sparing techniques are particularly marked, such that it would be advantageous to predict outcomes in advance to aid in making a clinical decision about how to treat the medical subject.

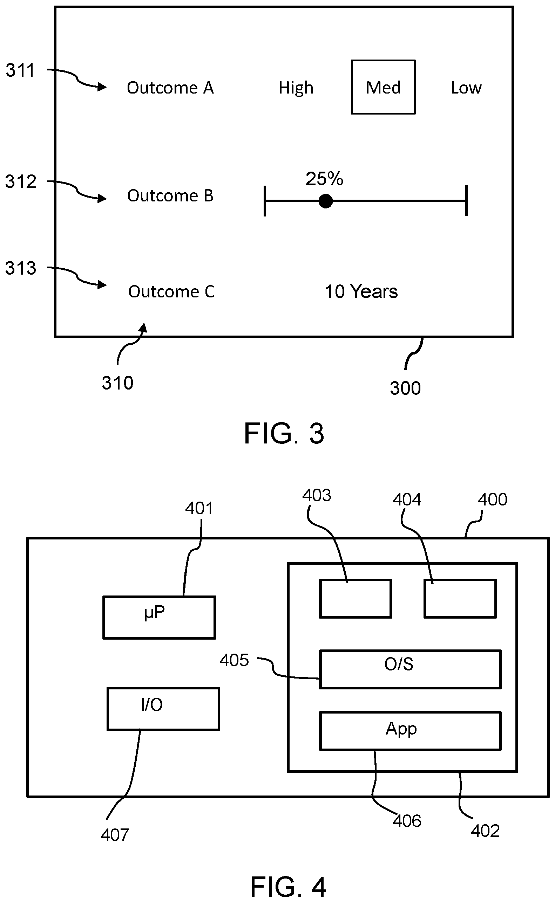

Fig. 3 illustrates a visual representation 310, provided by a user interface 300, of example predicted outcomes for performing one or more potential medical procedure on the medical subject. As previously explained, the potential medical procedure(s) are identified by a user at a user interface. Accordingly, the value(s) of the predicted outcome(s) will respond to changes in the identified potential medical procedure(s).

The examples of predicted outcomes may be subdivided into a number of outcome types, namely: (predicted) risk measures; and/or (predicted) functional outcomes.

A risk measure is a measure of a predicted risk to the medical subject. In the context of the present disclosure, a risk will indicate a risk of the occurrence of a clinically adverse effect.

The risk measure may be defined using binary, categorical and/or numeric data. For instance, a risk measure may be a binary value indicating whether the predicted risk is "High" or "Low". A categorical priority may indicate a discrete value (e.g., "High", "Medium" or "Low" risk). A numeric value for risk may be defined with respect to a predetermined scale. A predetermined scale may range from 0 to 1, 0 to 10, 1 to 10, 0 to 100 or 1 to 100, although other suitable scales will be apparent to the skilled person.

One example of a risk measures is patient-specific risk, which identifies a risk to the medical subject undergoing the procedure(s) (defined by the user-defined procedural information), e.g., independently of any oncology information. Such a risk measure may be generated by a risk model that processes the user-defined procedural information and only a portion of the subject information that does not include pathology or oncology information (e.g., subject information comprising only demographic, physiological, biological, family and/or genetic information).

Another example of a risk measure is an oncological risk, which identifies a risk to a generic patient having the oncology of the medical subject undergoing the procedure(s) (defined by the user-defined procedural information), e.g., independent of any non-oncology related subject information. Such a risk measure may be generated by a risk model that processes the user-defined procedural information and only a portion of the subject information that includes pathology or oncology information.

Another example of a risk measure is a risk of procedural complications. This may identify a risk of complications arising during the performance of the procedure(s) defined by the user-defined procedural information. Such a risk measure may be generated by a risk model that maps the combination of subject information and user-defined procedural information to known risks of procedural complications.

Fig. 3 provides an example of visual representation 311 of a risk measure. In the illustrated example, the risk measure (for an outcome "Outcome A") has a categorical data format. Other modifications will be apparent to the skilled person.

A predicted functional outcome may indicate a probability such as a recovery probability; a survival probability and/or a reoccurrence probability. Fig. 3 provides an example of visual representation 312 of a probability. In the illustrated example, the probability (for an outcome "Outcome B") is on a scale of from 0 to 100, although other scales will be apparent to the skilled person (e.g., 0 to 1 or 0 to 10).

A recovery probability identifies a probability of recovery of a biological function of the medical subject. A recovery probability may, for instance, identify a probability of a recovery within a particular time period (e.g., within 1 month, 3 months or 6 months) after the procedure(s) is/are performed.

The relevant biological function(s) may depend upon the purpose of the medical procedure(s). As a working example, if the potential procedure(s) are designed for prostatectomy, then suitable biological functions include: continence function; rectal function; and/or potency function. Other examples will be apparent to the skilled person.

A survival probability identifies a probability of survival of the medical subject (e.g., within a particular period of time after the medical procedure(s)). This may, for instance, comprise a probability of 10-year survival.

A reoccurrence probability identifies a probability of a medical condition or pathology reoccurring (e.g., within a particular period of time after the medical procedure(s)). More particularly, the reoccurrence probability may identify a probability of the medical condition for which the medical procedure(s) are designed to treat reoccurring. As a working example, if the medical procedure(s) are designed for treating prostate cancer, then the reoccurrence probability may identify a probability of biochemical recurrence (BCR) occurring within a particular time period after the medical procedure(s), e.g., within 1 year or within 5 years.

Another example of a predicted functional outcome is a predicted period of time. Fig. 3 provides an example of visual representation 313 of a predicted period of time. In the illustrated example, the predicted prior of time (for an outcome "Outcome C") is on a scale of years, although other scales will be apparent to the skilled person (e.g., months).

The predicted prior of time may represent a predicted recovery time, a predicted survival time or a predicted regain of function time. The predicted period of time may be generated in a similar manner to the probabilities previously described.

In some examples, the subject information comprises one or more radiology results and one or more pathology results.

The radiology result(s) and pathology result(s) are for a same clinical condition or disease (e.g., assessment of a tumor, cancer, or growth). A radiology result is a result or clinical assessment performed by a radiologist using imaging procedures (e.g., an MRI procedure, a CT procedure, an X-ray procedure, an ultrasound procedure and so on). A pathology result is a result or clinical assessment performed by a pathologist, e.g., from biopsy samples of the clinical condition or disease.

A result may define or identify one or more findings, e.g., identification of a lesion, growth, or the like in a region of interest (e.g., a prostate).

The present disclosure recognizes that a concordance or discordance between the radiology result(s) and the pathology result(s) can significantly affect treatment decision making by a clinician. However, it is also recognized that identifying discordance and concordance between the radiology result(s) and the pathology result(s) is challenging and time-consuming, particularly when there are multiple findings (e.g., multiple lesions).

Some proposed embodiments determine a level of concordance or similarly between the radiology result(s) and the pathology result(s) to aid in the making of a treatment decision by a clinician. The method may therefore further comprise determining one or more measures of similarity between radiology result(s) and pathology result(s).

In some examples, the determined one or more measures of similarity may, for instance, be visually represented at a user interface for the clinician. This facilitates improved understanding of any discordance between the two forms of result(s) to improve a clinical decision-making process.

In some examples, the determined one or more measures of similarity form part of the patient information processed to produce the predicted outcome(s). This ensures that concordance between radiology and pathology findings is taken into account when producing the predicted outcome(s), increasing the accuracy of the predicted outcomes.

In other examples, the determined one or more measures of similarity are used to determine a confidence of a predicted outcome for the medical subject. In particular, the less similar the radiology and pathology results, the less confidence in the predicted outcome. Approaches may therefore comprise determining a confidence of each predicted outcome using the one or more measures of similarity.

Approaches for determining one or more measures of similarity between radiology result and a pathology result is hereafter described.

In the context of such approaches, it is assumed that the radiology result comprises one or more radiology findings, each radiology finding having a location and a corresponding severity or measure of clinical significance (e.g., a PI-RADS score), which may be defined by the radiologist. Similarly, the pathology result comprises one or more pathological findings, each pathological finding having a location and a corresponding severity or measure of clinical significance (e.g., a Gleason score or Gleason Grade Group), which may be defined by the pathologist.

One measure of similarity is an overall measure of similarity. This overall measure may be determined by categorizing each radiology finding and each pathological finding, e.g., into at least three categories: "Clinically Significant", "Clinically Insignificant"; and "Benign". The decision as in which category a radiology/pathological finding lies may be responsive to the associated severity or measure of clinical significance, e.g., by determining into which of a plurality of predefined ranges the severity or measure falls. The overall measure may represent a measure of similarity or overlap between the categorization of the radiology findings and the pathological findings.

Another measure of similarity may be performed on a finding-by-finding basis. For instance, each of one or more pairs of radiology and pathological finding (where findings in a same pair have at a same location) may be compared to determine a corresponding number of finding measures of similarity. In particular, findings in a same pair may be categorized, e.g., using a previously described approach. If the findings in a same pair are in a same category, the finding measure of similarity indicates concordance, otherwise, the finding measure of similarity indicates discordance.

If one or more finding measures of similarity is displayed, then this may be provided on a visual representation of the investigated region of interest (e.g., prostate), with a location of each finding measure of similarity being positioned at the location of the corresponding pair of radiology and pathological findings.

Another example measure of similarity may be performed on a category basis. For instance, each radiology finding and each pathological finding may be categorized, e.g., using a previously described process. A category measure of similarity may be generated for each category, e.g., identifying a difference between the number of radiological findings and pathological findings in the category. The greater the difference, the less the similarity.

The present disclosure also proposes a computer-implemented method for determining one or more measures of similarity between a radiology result and a pathology result. The radiology result comprises one or more radiology findings, having a location and score of clinical significance. The pathology result comprises one or more pathological findings, having a location and score of clinical significance.

The method comprises processing the radiology finding(s) and the pathological finding(s) to determine one or more measures of similarity between the radiology result and the pathological result.

In preferred examples, the method comprises categorizing each radiology finding and each pathological finding using their respective scores of clinical significance. The method may comprise determining the one or more measures of similarity using the determined category of each radiology finding and each pathological finding.

As previously mentioned, a radiology result is a result or clinical assessment performed by a radiologist using imaging procedures (e.g., an MRI procedure, a CT procedure, an X-ray procedure, an ultrasound procedure and so on). A pathology result is a result or clinical assessment performed by a pathologist, e.g., from biopsy samples of the clinical condition or disease.

Previously described embodiments propose approaches for determining a measure of similarity between a radiology result and a pathology result of a single medical subject. Further examples may determine measures of similarity of radiology results and pathology results across a larger population of medical subject. This may, for instance, facilitate population-trends for differences or similarities between radiology results and pathology results. Any proposed approach may be employed, but further using additional radiology and pathology results.

It has previously been described how, in some embodiments, one or more machine-learning algorithms are used to process the subject information and the user-defined procedural information to produce the predicted outcome(s) of performing the identified potential medical procedure on the medical subject.

A machine-learning algorithm is any self-training algorithm that processes input data in order to produce or predict output data. Here, the input data comprises subject information and the user-defined procedural information and the output data comprises a predicted outcome of performing the identified potential medical procedure on the medical subject.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g., the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g., ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example instances of subject information and the user-defined procedural information. The training output data entries correspond to predicted outcome(s) of performing the identified potential medical procedure.

Fig. 4 illustrates an example of a processing system 400 within which one or more parts of an embodiment may be employed. Various operations discussed above may utilize the capabilities of the processing system 400. For example, one or more parts of a system for generating the predicted outcome(s) may be incorporated in any element, module, application, and/or component discussed herein. In this regard, it is to be understood that system functional blocks can run on a single processing system or may be distributed over several processing systems and locations (e.g., connected via internet).

The processing system 400 includes, but is not limited to, PCs, workstations, laptops, PDAs, palm devices, servers, storages, and the like. Generally, in terms of hardware architecture, the processing system 400 may include one or more processors 401, memory 402, and one or more I/O devices 407 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 401 is a hardware device for executing software that can be stored in the memory 402. The processor 401 can be virtually any custom made or commercially available processor, a central processing unit (CPU), a digital signal processor (DSP), or an auxiliary processor among several processors associated with the processing system 400, and the processor 401 may be a semiconductor-based microprocessor (in the form of a microchip) or a microprocessor.

The memory 402 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 402 may incorporate electronic, magnetic, optical, and/or other types of storage media. Note that the memory 402 can have a distributed architecture, where various components are situated remote from one another, but can be accessed by the processor 401.

The software in the memory 402 may include one or more separate programs, each of which comprises an ordered listing of executable instructions for implementing logical functions. The software in the memory 402 includes a suitable operating system (O/S) 405, compiler 404, source code 403, and one or more applications 406 in accordance with exemplary embodiments. As illustrated, the application 406 comprises numerous functional components for implementing the features and operations of the exemplary embodiments. The application 406 of the processing system 400 may represent various applications, computational units, logic, functional units, processes, operations, virtual entities, and/or modules in accordance with exemplary embodiments, but the application 406 is not meant to be a limitation.

The operating system 405 controls the execution of other computer programs, and provides scheduling, input-output control, file and data management, memory management, and communication control and related services. It is contemplated by the inventors that the application 406 for implementing exemplary embodiments may be applicable on all commercially available operating systems.

Application 406 may be a source program, executable program (object code), script, or any other entity comprising a set of instructions to be performed. When a source program, then the program is usually translated via a compiler (such as the compiler 404), assembler, interpreter, or the like, which may or may not be included within the memory 402, so as to operate properly in connection with the O/S 405. Furthermore, the application 406 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 407 may include input devices such as, for example but not limited to, a mouse, keyboard, scanner, microphone, camera, etc. Furthermore, the I/O devices 407 may also include output devices, for example but not limited to a printer, display, etc. Finally, the I/O devices 407 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 407 also include components for communicating over various networks, such as the Internet or intranet.

If the processing system 400 is a PC, workstation, intelligent device or the like, the software in the memory 402 may further include a basic input output system (BIOS) (omitted for simplicity). The BIOS is a set of essential software routines that initialize and test hardware at startup, start the O/S 405, and support the transfer of data among the hardware devices. The BIOS is stored in some type of read-only-memory, such as ROM, PROM, EPROM, EEPROM or the like, so that the BIOS can be executed when the processing system 400 is activated.

When the processing system 400 is in operation, the processor 401 is configured to execute software stored within the memory 402, to communicate data to and from the memory 402, and to generally control operations of the processing system 400 pursuant to the software. The application 406 and the O/S 405 are read, in whole or in part, by the processor 401, perhaps buffered within the processor 401, and then executed.

When the application 406 is implemented in software it should be noted that the application 406 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 406 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

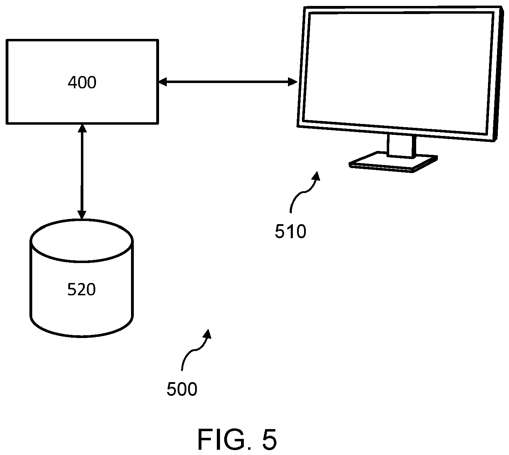

Fig. 5 illustrates a user interface system 500 according to a proposed embodiment.

The user interface system 500 comprise a processing system 400 and a user interface 510. The processing system is configured to perform the function of any herein disclosed method, and may be embodied as previously described.

The user interface is configured to provide or generate the user-defined procedural information. In particular, a user may be able to interact with one or more visual representations of possible procedures to be performed on the medical subject, to thereby select or otherwise identify the potential medical procedure(s) for which the predicted outcome(s) are to be generated.

The user interface 510 may also provide a visual representation of at least the predicted outcome(s).

The processing system 400 may obtain the subject information from a database or storage unit 520, which may form part of the user interface system.

The processing system 400 may obtain the user-defined procedural information from the user interface 510.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method of generating at least one predicted outcome of a medical procedure on a medical subject, the method comprising:
obtaining subject information that identifies one or more characteristics of the medical subject;
obtaining user-defined procedural information identifying a potential medical procedure to be performed on the medical subject, wherein the user-defined procedural information is defined by a user interacting with a user interface; and
processing the subject information and the user-defined procedural information using one or more risk models to generate at least one predicted outcome of performing the identified potential medical procedure on the medical subject.

2. The computer-implemented method of claim 1, wherein the user-defined procedural information identifies at least one potential surgical procedure to be performed on the medical subject.

3. The computer-implemented method of any one of claims 1-2, wherein the user-defined procedural information identifies at least one potential prostatectomy surgical procedure to be performed on the medical subject.

4. The computer-implemented method of any one of claims 1-3, wherein the user-defined procedural information identifies at least one potential nerve-sparing technique to be performed on the medical subject.

5. The computer-implemented method of any one of claims 1-4, wherein the at least one predicted outcome comprises one or more risk measures, each risk measure being a measure of predicted risk to the medical subject.

6. The computer-implemented method of claim 5, wherein the one or more risk measures comprise a measure of patient-specific risk; oncological risk; and/or risk of procedural complications.

7. The computer-implemented method of any one of claims 1-6, wherein the at least one predicted outcome comprises one or more predicted functional outcomes for the medical subject.

8. The computer-implemented method of claim 7, wherein the at least one predicted outcome comprises one or more recovery probabilities, each recovery probability identifying a probability of recovery of a biological function of the medical subject.

9. The computer-implemented method of any one of claims 7 or 8, wherein the at least one predicted outcome comprises one or more survival probabilities, each survival probability identifying a probability of survival of the medical subject, if the at least one potential procedure is performed on the medical subject, for a predetermined period of time after the at least one potential procedure is performed on the medical subject.

10. The computer-implemented method of any one of claims 1-9, wherein the at least one predicted outcome comprises one or more reoccurrence probabilities, each reoccurrence probability identifying a probability of a medical condition or pathology condition reoccurring, if the at least one potential procedure is performed on the medical subject, within a predefined period of time after the at least one potential procedure is performed on the medical subject.

11. The computer-implemented method of any one of claims 1-10, wherein the at one risk models uses one or more clinical guidelines to generate the at least one predicted outcome.

12. The computer-implemented method of any one of claims 1-11, wherein the at one risk models uses one or more machine-learning methods to generate the at least one predicted outcome.

13. The computer-implemented method of any of claims 1-12, further comprising controlling a user interface to provide a visual representation of the at least one predicted outcome.

14. A computer program comprising computer program code which, when executed on a computing device having a processing system, cause the processing system to perform all of the steps of the method according to any one of claims 1-13.

15. A processing system configured to generate at least one predicted outcome of a medical procedure on a medical subject, the processing system being configured to:
obtain subject information that identifies one or more characteristics of the medical subject;
obtain user-defined procedural information identifying a potential medical procedure to be performed on the medical subject, wherein the user-defined procedural information is defined by a user interacting with a user interface; and
process the subject information and the user-defined procedural information using one or more risk models to generate at least one predicted outcome of performing the identified potential medical procedure on the medical subject.
